# EUROPÄISCHE PATENTSCHRIFT

(11) **EP 2 990 076 B1**
(45) Veröffentlichungstag und Bekanntmachung des Hinweises auf die Patenterteilung: **14.12.2016**
(21) Anmeldenummer: 15173598.2
(22) Anmeldetag: 24.06.2015
(51) Int. Cl.: A61N 1/08, A61N 1/37, G01R 33/032, G01R 33/28

(54) **IMPLANTAT MIT EINER MRI-GERÄTEERKENNUNG**
IMPLANT WITH MRI DEVICE RECOGNITION
IMPLANT DOTÉ D'UNE RECONNAISSANCE D'APPAREIL MRI

(30) Priorität: 01.09.2014 US 201462044339 P
(43) Veröffentlichungstag der Anmeldung: 02.03.2016
(73) Patentinhaber: BIOTRONIK SE & Co. KG, 12359 Berlin (DE)
(72) Erfinder: Lippitz, Holger, 12205 Berlin (DE); Weiss, Ingo, 12435 Berlin (DE)
(74) Vertreter: Galander, Marcus

(56) Entgegenhaltungen:
- EP-A1- 2 338 559
- EP-A2- 2 338 564
- WO-A2-2013/055246
- DE-A1- 19 920 429
- US-A1- 2010 087 892
- US-B2- 6 462 539

## Beschreibung

Die offenbarte Technologie bezieht sich auf ein Implantat mit einer Magnetfelderkennung, insbesondere auf ein Implantat zur Erkennung von Feldern, die durch ein MRI-Gerät erzeugt werden.

Obwohl Untersuchungen mit MRI- (Magnetic Resonance Imaging, Kernspintomographie) Geräten einen immer höheren Stellenwert in der diagnostischen Medizin einnehmen, sind für einen nicht unerheblichen Teil der potentiellen Patienten die Untersuchungen mit MRI-Geräten kontraindiziert. Eine solche Kontraindikation kann zum Beispiel durch das Vorhandensein eines implantierten medizinischen Gerätes (IMD) gegeben sein.

Es existieren verschiedene Ansätze, um die Kompatibilität von implantierten medizinischen Geräten mit MRI-Geräten, genauer mit den Arbeitsbedingungen im Einflussbereich eines MRI-Gerätes, zu erhöhen. So wird versucht die für MRI-Geräte charakteristischen Felder zu detektieren, um daraus entsprechende Konsequenzen ziehen zu können.

Aus dem Stand der Technik sind verschiedene Methoden zur Detektion von Magnetfeldern und anderen elektromagnetischen Feldern bekannt.
So beschreibt die US 2008/0154342 A1 einen Magnetfelddetektor auf Basis eines GMR (Giant Magneto Resistance) Sensors oder eines Bandpassfilters in der Antennenschaltung.

Die US 2011/0152672 A1 beschreibt ein Implantat, bei dem ein elektrooptischer Wandler zur Detektion von für MRI-Geräte typische RF-/HF-Felder verwendet wird. Die EP 2 338 564 beschreibt ein Implantat gemäß der Präambel des Anspruchs 1.

Die US 6,462,539 B2 bezieht sich auf eine Vorrichtung zum Bestimmen von Magnetfeldern mittels eines optischen Aufbaus mit einem Doppelbrechungselement, das auf dem Faradayeffekt beruht.

Der beschriebene Stand der Technik bezieht sich jeweils auf eine Messung eines statischen Magnetfeldes oder die Messung von typischen RF-/HF-Feldern (im Folgenden wird unter HF auch RF subsumiert) oder eine Kombination von zwei Messmethoden zur Bestimmung sowohl der statischen Magnetfelder als auch der HF-Felder. Eine gleichzeitige Detektion der RF-/HF-Felder und der Magnetfelder in einer Messeinrichtung ist bisher nicht bekannt. Auch müssen die bisherigen optischen aufbauten für quantitative Aussagen aufwendig kalibriert werden oder bedürfen eines komplexen Aufbaus.

Es ist Aufgabe der Erfindung, ein Messsystem für ein Implantat bereitzustellen, das diese Mängel im Stand der Technik behebt.

Gelöst wird die Aufgabe durch ein Gerät nach dem unabhängigen Anspruch 1, ein implantierbares medizinisches Gerät (IMD) mit
- mindestens einer Spannungsquelle,
- mindestens einer Kontrolleinheit (110),
- mindestens einer Kommunikationsspule (120), und
- einem optischen Aufbau mit
   - mindestens einer Leuchtdiode (LED) (130, 140, 210),
   - mindestens einem Lichtdetektor (250),
   - mindestens einem ersten und einem zweiten Polarisationsfilter (220, 240),
   - mindestens einem Lichtleiter, der als Faradayelement (230', 230") ausgeführt ist, und wobei der optische Aufbau mindestens einen optischen Pfad enthält und wobei
   - die mindestens eine LED (130, 140, 210) mit der Kommunikationsspule derart elektronisch verbunden ist, dass die LED (130, 140, 210) Licht emittiert, wenn in die Kommunikationsspule ein HF-Feld eingekoppelt,
   - die LED (130, 140, 210) mit einer ersten Seite des ersten Polarisationsfilters (220) verbunden ist,
   - eine zweite Seite des ersten Polarisationsfilters (220) mit einem ersten Ende des Faradayelements (230', 230") verbunden ist,
   - eine erste Seite des zweiten Polarisationsfilters (240) mit einem zweiten Ende des Faradayelements (230', 230") verbunden ist,
   - eine zweite Seite des zweiten Polarisationsfilters (240) mit dem Lichtdetektor (250) verbunden ist,
   - das Faradayelement (230', 230") mindestens eine Biegung (260) aufweist und das Faradayelement (230', 230") durch die Biegung in mindestens zwei Abschnitte geteilt ist, und das Faradayelement (230', 230") in einem Formteil mit einer der Biegung (260) des Faradayelementes (230', 230") entsprechenden Biegung fest verankert ist.

Das Faradayelement kann dafür in ein Formteil eingelegt und/oder mit diesem verbunden werden und/oder in einer vorgegebenen Form von einem einen Feststoff bildenden Material umgeben, zum Beispiel umgossen, werden. Durch die Biegung wird erreicht, dass das Faradayelement auf Magnetfelder in einer Ebene empfindlich ist und so das statische Magnetfeld eines MRI-Gerätes erfassen kann. Eine Bestimmung des Magnetfeldes erfolgt nur in der durch das Faradayelement aufgespannten Ebene.
Das von dem Lichtdetektor detektierte Licht kann von einer Auswerteeinheit ausgewertet werden und ein entsprechendes Steuersignal generieren, das eine Umschaltung oder Beibehaltung eines Betriebsmodus des implantierten medizinischen Gerätes bewirkt. Das Beschriebene HF-Feld, das in die Kommunikationsspule einkoppelt oder einwirkt, ist vorzugsweise ein RF-Feld und besonders bevorzugt ein RF-Signal eines MRI-Gerätes, wobei die Frequenz des RF-Signals von der magnetischen Flussdichte des MRI-Gerätes abhängig ist.

Bevorzugt weist die mindestens eine Biegung zwischen den mindestens zwei Abschnitten des Faradayelementes einen Winkel von jeweils 90° auf. Der Winkel kann aber auch in Anpassung an das implantierbare medizinische Implantat einen anderen Winkel aufweisen, insbesondere einen an die Gehäuseform angepassten Winkel. Auch der Krümmungsradius kann an den Krümmungsradius des Gehäuses des implantierbaren medizinischen Gerätes angepasst sein. Alternativ können beim Vorhandensein von mehr als einer Biegung nur ein Winkel 90° betragen oder mehrere aber nicht alle Winkel 90° betragen. Besonders bevorzugt wird, dass die mindestens zwei Biegungen so gestaltet sind, dass das Faradayelement eine Längsausdehnung in allen drei Raumrichtungen hat, so dass eine Magnetfeldbestimmung nicht nur in einer Ebene, sondern im ganzen durch die drei Raumrichtungen aufgespannten Dreibein (Raum) möglich ist. Da Implantate in einer Raumrichtung meist wenig ausgedehnt sind, also flach sind, kann es bevorzugt sein, mehrere Teilabschnitte in dieser Richtung verlaufen zu lassen, also beispielsweise das Faradayelement im Zickzack oder mäandernd in dieser Raumrichtung zu verlegen, um eine ausreichende Strecke für das detektierbare Auftreten des Faradayeffektes bereitzustellen.

Auch wird bevorzugt, dass der optische Aufbau in einen Montagerahmen integriert ist, das Formteil also ganz oder teilweise Bestandteil eines Montagerahmens ist. Das Formteil kann aber auch ganz oder teilweise in den Montagerahmen eingefügt und so Bestandteil des Montagerahmens werden.

In einer bevorzugten Ausführungsform sind die Polarisationsfilter so eingestellt, dass ohne ein anliegendes Magnetfeld kein Licht durch den zweiten Polarisationsfilter austritt und kein Licht auf den Lichtdetektor fällt. Die Polarisationsfilter sind als gekreuzt eingestellt. Dabei wird berücksichtigt, dass die verwendeten optischen Elemente auch ohne das Vorhandensein eines Magnetfeldes einen Einfluss auf die Polarisationsrichtung des Lichtes haben können. Dieser Einfluss muss beim Aufbau kompensiert werden. Daher kann der Winkel zwischen den Polarisationsrichtungen von den Polarisationsfiltern von 90° Grad abweichen. Der Winkel kann von Aufbau zu Aufbau variieren, wichtig ist die Eigenschaft, dass kein oder nur ein Mindestmaß an Licht aus dem zweiten Polarisationsfilter austritt, die Polarisationsfilter also gekreuzt stehen, wenn kein Magnetfeld auf den Aufbau wirkt.

In einer weiteren Ausführungsform ist das Formteil aus einem lichtundurchlässigen Material und das Formteil umschließt das Faradayelement so, dass nur durch die beiden mit den Polarisationsfiltern verbundenen Seiten Licht in das Faradayelement eintreten kann. Dieser Aufbau reduziert Streulicht, das zu einem erhöhten Hintergrundrauschen führt. Damit wird die Empfindlichkeit und Genauigkeit der Magnetfeldbestimmung erhöht. Auch wird bevorzugt, dass der gesamte optische Aufbau mit einem lichtundurchlässigen Kunststoff derart umgossen wird, dass kein Streulicht in den optischen Aufbau einkoppeln kann. Wie bei dem Formteil aus einem lichtundurchlässigen Material wird Streulicht in dem optischen Aufbau minimiert. Beide Maßnahmen zur Streulichtreduzierung können auch kombiniert werden.

In einer besonderen Ausführungsform weist das Faradayelement eine Verdetkonstante von mindestens 2 rad pro (Tesla und Meter) im Bereich des von der LED emittierten Lichtspektrums oder eines Teils des emittierten Lichtspektrums auf.

Auch wird bevorzugt, dass das Faradayelement aus einer Glasfaser besteht. Insbesondere kann das Faradayelement auch aus einer getemperten, also einer bei erhöhter Temperatur gelagerten und langsam abgekühlten, Glasfaser gebildet werden. Insbesondere kann der Tempervorgang vorgenommen werden, wenn die Glasfaser bereits in der später angestrebten Form, vor allem in Bezug auf die Biegung oder die Biegungen, gebracht wurde.

In einer bevorzugten Ausführungsform weist das Faradayelement mindestens eine weitere Biegung auf, so dass das Faradayelement in allen drei Raumrichtungen ein Magnetfeld detektieren kann. Besonders bevorzugt wird dabei die Ausführung der dann mindestens drei Faradayelemente als ein orthogonales Dreibein. Da bei herkömmlichen implantierbaren medizinischen Geräten eine Raumausdehnung in der Regel deutlich kürzer ist als in die anderen beiden, also analog zu einem flachen Quader, ist es sinnvoll, das Faradayelement in dieser kürzeren Raumrichtung mindestens einmal, oder aber auch mehrmals hin und her zurückzuführen, so dass die Gesamtlänge in dieser kürzeren Raumrichtung in etwa der Länge in die anderen Raumrichtungen entspricht.

Auch wird bevorzugt, dass der Lichtdetektor mit einer Auswerteeinheit verbunden ist, die anhand der Intensitätsänderung des detektierten Lichtes bestimmt, ob das anliegende Magnetfeld eine magnetische Flussdichte kleiner oder gleich 1 Tesla aufweist, oder 1,5 Tesla oder 3 Tesla oder 7 Tesla aufweist.

Besonders bevorzugt wird, dass die Auswerteeinheit in Abhängigkeit von der bestimmten Magnetfeldstärke bzw. der magnetischen Flussdichte ein Steuersignal generiert. Dadurch ist das implantierte medizinische Gerät mit der Auswerteeinheit in der Lage, unterschiedliche Schlüsse aus dem jeweils detektierten Licht zu ziehen. So können beispielsweise unterschiedliche Betriebsmodi für das implantierte medizinische Gerät durch unterschiedliche Detektionsergebnisse initiiert werden.

Eine weitere Ausführungsform zeichnet sich dadurch aus, dass ein weiterer optischer Pfad von der LED zu dem Lichtdetektor oder einen weiteren Lichtdetektor führt, wobei der weitere optische Pfad so ausgelegt wird, dass er als Referenz verwendet werden kann, ohne von einem Magnetfeld beeinflusst zu werden. Dieser weitere optische Pfad kann beispielsweise durch einen Lichtleiter mit einer sehr geringen Verdetkonstanten im Bereich des von der LED emittierten Lichtspektrums oder eines Teils des emittierten Lichtspektrums realisiert werden.

Besonders bevorzugt wird , dass der weitere optische Pfad zu einem weiteren Lichtdetektor führt und das die Lichtdetektoren mit einer Auswerteeinheit verbunden sind, die anhand der Intensitätsänderung des detektierten Lichtes bestimmt, ob das anliegende Magnetfeld eine magnetische Flussdichte kleiner oder gleich 1 Tesla aufweist, oder 1,5 Tesla oder 3 Tesla oder 7 Tesla aufweist. Dabei verwendet die Auswerteeinheit den weiteren optischen Pfad als Referenzpfad zur Bestimmung der Magnetfeldstärke bzw. der magnetischen Flussdichte.

In einer bevorzugten Ausführungsform ist die LED zusätzlich mit der Spannungsquelle oder einer weiteren Spannungsquelle derart verbunden, also vorgespannt, dass eine geringere HF-Einkopplung als ohne die Verbindung mit der Spannungsquelle oder der weiteren Spannungsquelle bereits ausreicht, dass die LED Licht emittiert. Dadurch wird das System sensitiver für die HF-Felder. Auch wird dadurch die Lichtausbeute erhöht und damit die Messungenauigkeit reduziert.

Bevorzugt wird auch, dass die LED durch eine Strombegrenzung geschützt wird. Zusätzlich erreicht man dadurch eine Begrenzung der maximalen Lichtintensität, wodurch eine Kalibrierung des Systems zur Bestimmung der Magnetfeldstärke bzw. der magnetischen Flussdichte möglich ist. Insbesondere ist dies möglich, wenn die in die Kommunikationsspule induzierten HF-Felder ebenfalls ausgewertet und von der Auswerteeinheit in die Magnetfeldbestimmung einbezogen werden. Der Begriff Magnetfeldbestimmung bezieht sich durchgehend auf die magnetische Flussdichte in Tesla, da dies die verbreitete Angabe bei MRI-Geräten ist.

Auch wird bevorzugt, dass die Vorspannung an der LED knapp unterhalb der Öffnungsgrenze der LED liegt.

In einer weiteren Ausführungsform ist die LED durch mindestens einen Kondensator an mindestens einer Seite von Gleichstromanteilen abgekoppelt.

Im Folgenden wird die Erfindung an Hand von Figuren beschrieben.
- Fig. 1: zeigt schematisch den Faraday-Effekt,
- Fig. 2: zeigt die Kommunikationsspule mit zwei Leuchtdioden und einer Kontrolleinheit,
- Fig. 3: zeigt einen erfindungsgemäßen optischen Aufbau mit einer Biegung.

### Detaillierte Beschreibung der Erfindung

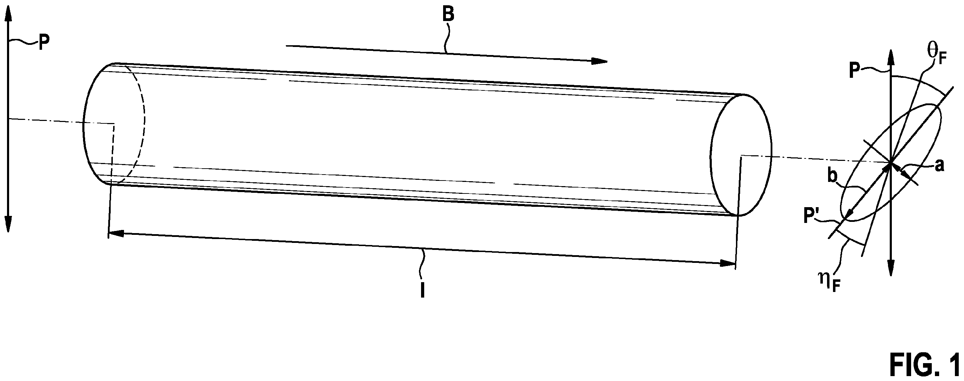

Die Figur 1 zeigt schematisch die Funktionsweise eines Faradayelements, wobei P die ursprüngliche Polarisationsrichtung ist, B die Richtung des Magnetfeldes zeigt, 1 die Länge des Faradayelementes ist, P' die resultierende Polarisationsrichtung ist, a und b die große und kleine Halbachse des resultierenden Ellipsoiden sind und Theta F die Faraday-Rotation oder der Drehwinkel der Polarisation ist und Eta F die Faraday Ellipsität ist.

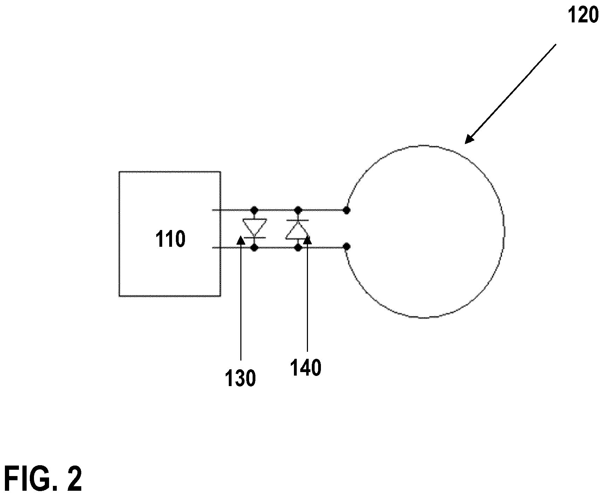

Die Figur 2 zeigt einen Teil des Schaltbilds eines Implantats mit einer Spannungsquelle, einer Kontrolleinheit 110 und einer Kommunikationsspule 120, die mit mindestens einer LED, bevorzugt mit zwei LEDs 130, 140 mit der Kommunikationsspule elektrisch so verbunden ist, dass die mindestens eine LED Licht emittiert, wenn über die Kommunikationsspule ein für MRI-Geräte typisches HF-Feld eingekoppelt wird. Die Kontrolleinheit 110 verbindet die Kommunikationsspule mit anderen Komponenten des implantierbaren medizinischen Implantats.

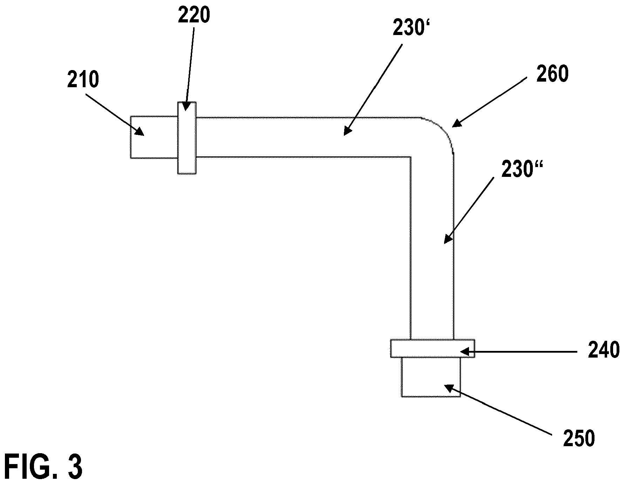

Die Figur 3 zeigt schematisch den optischen Aufbau. Die LED 210 ist über einen optischen Aufbau mit einem Lichtdetektor 250, z.B. einer Photodiode, verbunden. Weitere Bestandteile des optischen Aufbaus sind ein Polarisationsfilter 220, darauf folgend mindestens ein Faradayelement 230', 230", z.B. dotierter Glasstab mit hoher Verdetkonstante oder bevorzugt eine Glasfaser mit hoher Verdetkonstante, besonders bevorzugt eine unter Biegung polarisationserhaltene Glasfaser mit einer hohen Verdetkonstante, zum Beispiel eine getemperte Glasfaser. An das Faradyelement 230', 230" schließt ein weiterer Polarisationsfilter 240 an, der zum ersten um 90° verdreht, also gekreuzt ist. Für den Fall, dass eine Glasfaser verwendet wird, und insbesondere wenn diese Glasfaser nicht geradlinig verläuft, kann die Glasfaser bereits ohne ein Anliegen eines Magnetfeldes eine Drehung der Polarisationsrichtung oder eine Depolarisation bewirken, weshalb der Winkel zwischen den Polarisationsfiltern von 90° abweichen kann, um das Passieren des Lichts durch den zweiten Filter zu verhindern. Der Lichtdetektor 250 ist mit der Kontrolleinheit 110 oder einer Auswerteeinheit (nicht gezeigt) verbunden und kann durch die Kontrolleinheit oder die Auswerteeinheit oder direkt mit der Spannungsquelle oder einer weiteren Spannungsquelle verbunden sein. Der Lichtdetektor 250 sendet ein Signal an die Kontrolleinheit 110, wenn er Licht über einer vorbestimmbaren Intensität detektiert. Das Signal kann ein konstantes Signal sein oder aber von der detektierten Intensität abhängen. Alternativ kann auch eine Auswerteeinheit ein Spannungs- und/oder Stromsignal vom Lichtdetektor 250 auslesen und weiterverarbeiten oder an die Kontrolleinheit 110 übermitteln.

Bei einer Verdetkonstante von 5,7 rad * T-1 m-1 für SiO2 bei 2,27 eV ergibt sich bei einer Länge einer Glasfaser als Faradayelement 230', 230" von 5 cm und einer Magnetfeldstärke von 1 Tesla eine Faraday-Rotation von etwa 16°, das heißt die Polarisationsachse des Lichts wird bei der Passage der Glasfaser in einem Magnetfeld mit der Stärke von einem Tesla um 16° geneigt, wodurch die Polarisationsfilter 220, 240 unter diesen Bedingungen für das Licht nicht mehr gekreuzt sind und so ein Anteil des Lichtes den zweiten Polarisationsfilter 240 passieren kann und so auf dem Lichtdetektor 250 nachweisbar ist.

Um zu verhindern, dass Streulicht in den optischen Aufbau eingekoppelt wird, kann dieser mit einem Gehäuse versehen werden, insbesondere kann dieses Gehäuse ganz oder teilweise in einem Montagerahmen integriert sein oder ganz oder teilweise Bestandteil eines solchen Montagerahmens sein.

Um den optischen Aufbau möglichst stabil zu gestalten, können die Elemente des optischen Aufbaus (LEDs, Polarisationsfilter, Faraday-Element & Photodetektor) in einem Bauteil fest und gegeneinander unbeweglich verbunden sein. Insbesondere können die Bauteile des optischen Aufbaus in einem Kunststoff vergossen oder mit einem Kunststoff umgossen werden.

Figur 3 zeigt auch ein Beispiel für ein Faradayelement 230', 230", das eine Biegung 260 von 90° aufweist. Dadurch wird das Faradayelement 230', 230" in zwei Bereiche 230' und 230" aufgeteilt, die jeweils für eine andere Komponente des Magnetfeldes in einer Ebene empfindlich sind. Dadurch lässt sich das Magnetfeld unabhängig von seiner Lage in der so von den beiden Faradayelementabschnitten aufgespannten Ebene detektieren.
Die durch die zwei Faradayelemente 230', 230" aufgespannte Ebene liegt dabei bevorzugt parallel zu der Gehäuseseite des Implantats, die die größte Fläche aufweist.

Die beiden Faradayelementabschnitte 230', 230" können durch eine Glasfaser realisiert werden, die eine Biegung 260 aufweist. Die Polarisationsfilter werden so zueinander gedreht, dass ohne ein anliegendes Magnetfeld kein Licht den zweiten Polarisationsfilter 240 passieren kann.

Es können auch drei Faradayelementabschnitte verwendet werden, die einen Raum aufspannen und so Magnetfelder in allen drei Raumrichtungen detektiert werden können, insbesondere können diese drei Faradayelementabschnitte durch eine Glasfaser mit zwei Biegungen realisiert werden.

Zusätzlich zum beschriebenen Aufbau kann das Licht der mindestens einen LED 130, 140, 210 auch auf einem weiteren optischen Pfad ohne ein Faradayelement zu einem weiteren Lichtdetektor geleitet werden und so als Referenz zur Verfügung stehen. Die Referenz kann dazu genutzt werden, um auf die quantitative Drehung der Polarisation und damit auf die Stärke des Magnetfeldes schließen zu können. Die Intensität des detektierten Lichtes hängt einerseits von der Größe der Drehung der Polarisation ab (der zweite Polarisationsfilter 240 lässt das Licht mit einem Faktor vom Sinus des Drehwinkels der Polarisation passieren) und von der Magnetfeldstärke, die die Drehung der Polarisationsrichtung im Faradayelement verursacht (Drehwinkel = Magnetfeldstärke in Tesla mal Verdetkonstante mal Wegstrecke im Faraday-Element parallel zum Magnetfeld). Bei der Betrachtung ist die Abschwächung der Lichtintensität durch die verwendeten optischen Bauteile zu berücksichtigen. Die Information über die Magnetfeldstärke kann zu einer Unterscheidung von unterschiedlichen MRI-Gerätetypen (1 Tesla, 1,5 Tesla, 3 Tesla, 7 Tesla) verwendet werden.

## Patentansprüche

1. Implantierbares medizinisches Gerät (IMD) mit
- mindestens einer Spannungsquelle,
- mindestens einer Kontrolleinheit (110),
- mindestens einer Kommunikationsspule (120), und
- einem optischen Aufbau mit
- mindestens einer Leuchtdiode (LED) (130, 140, 210),
- mindestens einem Lichtdetektor (250),
- mindestens einem Lichtleiter, und wobei der optische Aufbau mindestens einen optischen Pfad enthält und wobei
- die mindestens eine LED (130, 140, 210) mit der Kommunikationsspule derart elektronisch verbunden ist, dass die LED (130, 140, 210) Licht emittiert, wenn in die Kommunikationsspule ein HF-Feld eingekoppelt wird,
**dadurch gekennzeichnet, dass**
- die LED (130, 140, 210) mit einer ersten Seite des ersten Polarisationsfilters (220) verbunden ist,
- der Lichtleiter als Faradayelement (230', 230") ausgeführt ist,
- eine zweite Seite des ersten Polarisationsfilters (220) mit einem ersten Ende des Faradayelements (230', 230") verbunden ist,
- eine erste Seite des zweiten Polarisationsfilters (240) mit einem zweiten Ende des Faradayelements (230', 230") verbunden ist,
- eine zweite Seite des zweiten Polarisationsfilters (240) mit dem Lichtdetektor (250) verbunden ist,
- das Faradayelement (230', 230") mindestens eine Biegung (260) aufweist und das Faradayelement (230', 230") durch die Biegung in mindestens zwei Abschnitte geteilt ist, und
- das Faradayelement (230', 230") in einem Formteil mit einer der Biegung (260) des Faradayelementes (230', 230") entsprechenden Biegung fest verankert ist.

2. Implantierbares medizinisches Gerät nach Anspruch 1, **dadurch gekennzeichnet, dass** der durch die mindestens eine Biegung (260) zwischen den mindestens zwei Abschnitten des Faradayelementes (230', 230") erzeugte Winkel jeweils 90° beträgt.

3. Implantierbares medizinisches Gerät nach Anspruch 1 oder 2, **dadurch gekennzeichnet, dass** die Polarisationsfilter (220, 240) so eingestellt sind, dass ohne ein anliegendes Magnetfeld kein Licht durch den zweiten Polarisationsfilter (240) austritt und kein Licht auf den Lichtdetektor (250) fällt.

4. Implantierbares medizinisches Gerät nach einem der vorhergehenden Ansprüche, **dadurch gekennzeichnet, dass** das Formteil aus einem lichtundurchlässigen Material ist und dass das Formteil das Faradayelement (230', 230") so umschließt, dass nur durch die mit den Polarisationsfiltern (220, 240) verbundenen Seiten Licht in das Faradayelement (230', 230") eintreten kann.

5. Implantierbares medizinisches Gerät nach einem der vorhergehenden Ansprüche, **dadurch gekennzeichnet, dass** der gesamte optische Aufbau mit einem lichtundurchlässigen Kunststoff derart umgossen ist, dass kein Streulicht in den optischen Aufbau einkoppeln oder einwirken kann.

6. Implantierbares medizinisches Gerät nach einem der vorhergehenden Ansprüche, **dadurch gekennzeichnet, dass** das Faradayelement (230', 230") eine Verdetkonstante von mindestens 2 rad pro (Tesla und Meter) im Bereich des von der LED (130, 140, 210) emittierten Lichtspektrums oder eines Teils des emittierten Lichtspektrums aufweist.

7. Implantierbares medizinisches Gerät nach einem der vorhergehenden Ansprüche, **dadurch gekennzeichnet, dass** das Faradayelement (230', 230") aus einer Glasfaser besteht.

8. Implantierbares medizinisches Gerät nach einem der vorhergehenden Ansprüche, **dadurch gekennzeichnet, dass** das Faradayelement (230', 230") mindestens eine zweite Biegung aufweist, so dass das Faradayelement (230', 230") in allen drei Raumrichtungen ein Magnetfeld detektieren kann.

9. Implantierbares medizinisches Gerät nach einem der vorhergehenden Ansprüche, **dadurch gekennzeichnet, dass** der Lichtdetektor (250) mit einer Auswerteeinheit verbunden ist, die anhand der Intensitätsänderung des detektierten Lichtes bestimmt, ob das anliegende Magnetfeld eine magnetische Flussdichte kleiner oder gleich 1 Tesla aufweist oder 1,5 Tesla oder 3 Tesla oder 7 Tesla aufweist.

10. Implantierbares medizinisches Gerät nach Anspruch 9, **dadurch gekennzeichnet, dass** die Auswerteeinheit in Abhängigkeit von der bestimmten magnetischen Flussdichte ein Steuersignal generiert.

11. IMD nach einem der vorhergehenden Ansprüche, **dadurch gekennzeichnet, dass** ein weiterer optischer Pfad von der LED (130, 140, 210) zu dem mindestens einen Lichtdetektor (250) oder einen weiteren Lichtdetektor führt, wobei der weitere optische Pfad so ausgelegt ist, dass er als Referenzpfad verwendbar ist, ohne dass die Intensität des von der LED (130, 140, 210) emittierten Lichtes von einem Magnetfeld beeinflussbar ist.

12. Implantierbares medizinisches Gerät nach Anspruch 11, **dadurch gekennzeichnet, dass** der weitere optische Pfad zu mindestens einem weiteren Lichtdetektor führt und dass die Lichtdetektoren mit der mindestens einen Auswerteeinheit verbunden sind, die anhand der Intensitätsänderung des detektierten Lichtes bestimmt, ob die magnetische Flussdichte des anliegenden Magnetfeldes kleiner oder gleich 1 Tesla ist, oder 1,5 Tesla oder 3 Tesla oder 7 Tesla ist.

13. Implantierbares medizinisches Gerät nach einem der vorhergehenden Ansprüche, **dadurch gekennzeichnet, dass** die LED (130, 140, 210) zusätzlich mit der Spannungsquelle oder einer weiteren Spannungsquelle derart verbunden ist, dass eine geringere HF-Einkopplung als ohne die Verbindung mit der Spannungsquelle oder der weiteren Spannungsquelle bereits ausreicht, dass die LED (130, 140, 210) Licht emittiert.

14. Implantierbares medizinisches Gerät nach Anspruch 13, **dadurch gekennzeichnet, dass** durch die Verbindung mit der Spannungsquelle oder der weiteren Spannungsquelle eine Vorspannung an der LED (130, 140, 210) anliegt, die unterhalb der Öffnungsgrenze der LED (130, 140, 210) liegt.

15. Implantierbares medizinisches Gerät nach einem der vorhergehenden Ansprüche, **dadurch gekennzeichnet, dass** die LED (130, 140, 210) durch mindestens einen Kondensator an mindestens einer Zuleitung von der Kommunikationsspule zu der LED (130, 140, 210) von Gleichstromanteilen abgekoppelt ist.

## Claims

1. An implantable medical device (IMD) comprising
- at least one voltage source,
- at least one control unit (110),
- at least one communication coil (120), and
- an optical structure, with
- at least one light-emitting diode (LED) (130, 140, 210),
- at least one light detector (250),
- at least one optical fibre, and wherein the optical structure contains at least one optical path, and wherein
- the at least one LED (130, 140, 210) is electronically connected to the communication coil in such a way that the LED (130, 140, 210) emits light when an HF field is coupled into the communication coil,
**characterised in that**
- the optical structure is provided with at least one first and one second polarisation filter (220, 240),
- the optical fibre is formed as a Faraday element (230', 230"),
- the LED (130, 140, 210) is connected to a first side of the first polarisation filter (220),
- a second side of the first polarisation filter (220) is connected to a first end of the Faraday element (230', 230"),
- a first side of the second polarisation filter (240) is connected to a second end of the Faraday element (230', 230"),
- a second side of the second polarisation filter (240) is connected to the light detector (250),
- the Faraday element (230', 230") has at least one bend (260) and the Faraday element (230', 230") is divided by the bend into at least two portions, and
- the Faraday element (230', 230") is fixedly anchored in a shaped part with a bend corresponding to the bend (260) of the Faraday element (230', 230").

2. The implantable medical device as claimed in claim 1, **characterised in that** the angle produced by the at least one bend (260) between the at least two portions of the Faraday element (230', 230") is 90° in each case.

3. The implantable medical device as claimed in claim 1 or 2, **characterised in that** the polarisation filters (220, 240) are set such that, without the presence of a magnetic field, no light exits through the second polarisation filter (240) and no light impinges on the light detector (250).

4. The implantable medical device as claimed in any one of the preceding claims, **characterised in that** the shaped part is made of a light-impermeable material, and **in that** the shaped part surrounds the Faraday element (230', 230"), such that light can enter the Faraday element (230', 230") only through the sides associated with the polarisation filters (220, 240).

5. The implantable medical device as claimed in any one of the preceding claims, **characterised in that** the entire optical structure is cast around with a light-impermeable plastic in such a way that no stray light can be coupled into or influence the optical structure.

6. The implantable medical device as claimed in any one of the preceding claims, **characterised in that** the Faraday element (230', 230") has a Verdet constant of at least 2 rad per (tesla and meter) in the range of the light spectrum emitted by the LED (130, 140, 210) or part of the emitted light spectrum.

7. The implantable medical device as claimed in any one of the preceding claims, **characterised in that** the Faraday element (230', 230") consists of a glass fibre.

8. The implantable medical device as claimed in any one of the preceding claims, **characterised in that** the Faraday element (230', 230") has at least one second bend, such that the Faraday element (230', 230") can detect a magnetic field in all three spatial directions.

9. The implantable medical device as claimed in any one of the preceding claims, **characterised in that** the light detector (250) is connected to an evaluation unit which determines, on the basis of the intensity change of the detected light, whether the magnetic field present has a magnetic flux density less than or equal to 1 tesla, or 1.5 tesla or 3 tesla or 7 tesla.

10. The implantable medical device as claimed in claim 9, **characterised in that** the evaluation unit generates a control signal depending on the determined magnetic flux density.

11. The implantable medical device as claimed in any one of the preceding claims, **characterised in that** a further optical path leads from the LED (130, 140, 210) to the at least one light detector (250) or a further light detector, wherein the further optical path is configured such that it can be used as a reference path without it being possible for the intensity of the light emitted by the LED (130, 140, 210) to be influenced by a magnetic field.

12. The implantable medical device as claimed in Claim 11, **characterised in that** the further optical path leads to at least one further light detector, and **in that** the light detectors are connected to the at least one evaluation unit, which determines, on the basis of the intensity change of the detected light, whether the magnetic field present has a magnetic flux density less than or equal to 1 tesla, or 1.5 tesla or 3 tesla or 7 tesla.

13. The implantable medical device as claimed in any one of the preceding claims, **characterised in that** the LED (130, 140, 210) is additionally connected to the voltage source or a further voltage source in such a way that even a lower HF coupling than without the connection to the voltage source or the further voltage source is sufficient for the LED (130, 140, 210) to emit light.

14. The implantable medical device as claimed in claim 13, **characterised in that**, due to the connection to the voltage source or the further voltage source, a bias is applied across the LED (130, 140, 210) and is below the opening limit of the LED (130, 140,210).

15. The implantable medical device as claimed in any one of the preceding claims, **characterised in that** the LED (130, 140, 210) is decoupled from direct current components by at least one capacitor on at least one feed line from the communication coil to the LED (130, 140, 210).

## Revendications

1. Appareil médical implantable (IMD) avec
- au moins une source de tension,
- au moins une unité de contrôle (110),
- au moins une bobine de communication (120), et
- un montage optique avec
- au moins une diode lumineuse (LED) (130, 140, 210),
- au moins un détecteur de lumière (250),
- au moins une fibre optique, et où le montage optique contient au moins un trajet optique et où
- l'au moins une LED (130, 140, 210) est reliée avec la bobine de communication de manière électronique de sorte que la LED (130, 140, 210) émet une lumière lorsqu'un champ HF est couplé dans la bobine de communication,
**caractérisé en ce que**
- que le montage optique est équipé avec au moins un premier et un deuxième filtre de polarisation (220, 240),
- la fibre optique est conçue sous la forme d'un élément de Faraday (230', 230"),
- la LED (130, 140, 210) est reliée avec un premier côté du premier filtre de polarisation (220),
- un deuxième côté du premier filtre de polarisation (220) est relié avec une première extrémité de l'élément de Faraday (230', 230"),
- un premier côté du deuxième filtre de polarisation (240) est relié avec une deuxième extrémité de l'élément de Faraday (230', 230"),
- un deuxième côté du deuxième filtre de polarisation (240) est relié avec le détecteur de lumière (250),
- l'élément de Faraday (230', 230") présente au moins un coude (260) et l'élément de Faraday (230', 230") est divisé en moins deux segments par le coude, et
- l'élément de Faraday (230', 230") est ancré solidement dans un moule avec un coude correspondant au coude (260) de l'élément de Faraday (230', 230").

2. Appareil médical implantable selon la revendication 1, **caractérisé en ce que** l'angle généré par l'au moins un coude (260) entre les au moins deux segments de l'élément de Faraday (230', 230") est respectivement de 90 °.

3. Appareil médical implantable selon la revendication 1, ou 2, **caractérisé en ce que** les filtres de polarisation (220, 240) sont réglés de telle manière que, sans un champ magnétique appliqué, il n'y a pas de lumière sortant par le deuxième filtre de polarisation (240) et aucune lumière n'atteint le détecteur de lumière (250).

4. Appareil médical implantable selon l'une des revendications précédentes, **caractérisé en ce que** le moule est constitué d'un matériau transparent et que le moule entoure l'élément de Faraday (230', 230") de telle manière que de la lumière peut entrer uniquement dans l'élément de Faraday (230', 230") par les côtés reliés avec les filtres de polarisation (220, 240).

5. Appareil médical implantable selon l'une des revendications précédentes, **caractérisé en ce que** le montage optique complet est entouré en étant coulé dans une matière plastique transparente de telle sorte qu'aucune lumière diffusée ne peut se coupler ou agir dans le montage optique.

6. Appareil médical implantable selon l'une des revendications précédentes, **caractérisé en ce que** l'élément de Faraday (230', 230") présente une constante de Verdet d'au moins 2 rad par (Tesla et mètre) dans la région du spectre de lumière émis par la LED (130, 140, 210) ou dans une partie du spectre de lumière émis.

7. Appareil médical implantable selon l'une des revendications précédentes, **caractérisé en ce que** l'élément de Faraday (230', 230") est constitué d'une fibre de verre.

8. Appareil médical implantable selon l'une des revendications précédentes, **caractérisé en ce que** l'élément de Faraday (230', 230") présente au moins un deuxième coude de sorte que l'élément de Faraday (230', 230") peut détecter un champ magnétique dans toutes les trois directions spatiales.

9. Appareil médical implantable selon l'une des revendications précédentes, **caractérisé en ce que** le détecteur de lumière (250) est relié avec une unité d'exploitation, qui, à l'aide de la modification d'intensité de la lumière détectée, détermine si le champ magnétique appliqué présente une densité de flux magnétique inférieure ou égale à 1 Tesla, ou 1,5 Tesla, ou 3 Tesla, ou 7 tesla.

10. Appareil médical implantable selon la revendication 9, **caractérisé en ce que** l'unité d'exploitation génère un signal de commande en fonction de la densité de flux magnétique déterminée.

11. Appareil médical implantable selon l'une des revendications précédentes, **caractérisé en ce qu'**un autre trajet optique mène de la LED (130, 140, 210) vers l'au moins un détecteur de lumière (250) ou un autre détecteur de lumière, où l'autre trajet optique est conçu de sorte à ce qu'il soit utilisable en tant que trajet de référence sans que l'intensité de la lumière émise par la LED (130, 140, 210) ne soit influençable par un champ magnétique.

12. Appareil médical implantable selon la revendication 11, **caractérisé en ce que** l'autre trajet optique mène vers au moins un autre détecteur de lumière et que les détecteurs de lumière sont reliés avec l'au moins une unité d'exploitation, qui, à l'aide de la modification d'intensité de la lumière détectée, détermine si la densité de flux magnétique du champ magnétique appliqué est inférieure ou égale à 1 Tesla, ou 1,5 Tesla, ou 3 Tesla, ou 7 Tesla.

13. Appareil médical implantable selon l'une des revendications précédentes, **caractérisé en ce que** la LED (130, 140, 210) est reliée de manière complémentaire avec la source de tension ou une autre source de tension de telle manière qu'un faible couplage HF suffit déjà pour que la LED (130, 140, 210) émette de la lumière sans la liaison avec la source de tension ou avec l'autre source de tension.

14. Appareil médical implantable selon la revendication 13, **caractérisé en ce que** la liaison avec la source de tension ou avec l'autre source de tension applique une prétension sur la LED (130, 140, 210), qui se situe en dessous de la limite d'ouverture de la LED (130, 140, 210).

15. Appareil médical implantable selon l'une des revendications précédentes, **caractérisé en ce que** la LED (130, 140, 210) est découplée de composantes de courant continu sur au moins une ligne d'alimentation de la bobine de communication vers la LED (130, 140, 210) par au moins un condensateur.
